# EUROPEAN PATENT APPLICATION

(11) **EP 3 805 242 A1**
(43) Date of publication of application: **14.04.2021**
(21) Application number: 19201713.5
(22) Date of filing: 07.10.2019
(51) Int. Cl.: C07H 1/00, C07H 21/04, C12N 15/113, A61K 31/7088, A61P 11/06

(54) **METHOD FOR THE PRODUCTION OF A CATALYTICALLY ACTIVE DNA MOLECULE HAVING IMPROVED ACTIVITY AND ITS USE IN A METHOD OF TREATING ASTHMA**

(71) Applicant: Sterna Biologicals GmbH & Co. KG, 35037 Marburg (DE)
(72) Inventor: Rupp, Thomas, 35037 Marburg (DE)
(74) Representative: V.O.

(57) **Abstract**

The present invention refers to a method for the production of a catalytically active DNA molecule resulting in a significantly decreased amount of impurities in the catalytically active DNA molecule, to a catalytically active DNA molecule obtainable by such method and a pharmaceutical composition comprising such catalytically active DNA molecule as well as their use in a method for the prevention and/or treatment of a GAT-3-driven disease.

## Description

The present invention refers to a method for the production of an oligonucleotide including a catalytically active DNA, to the oligonucleotide obtainable by this method, to a pharmaceutical composition comprising such oligonucleotide, and the use of the catalytically active DNA or pharmaceutical composition in a method of treating aGATA-3 driven disease, wherein the oligonucleotide comprises a low amount of impurities.

### Technical background

A catalytically active DNA molecule is a single-stranded, synthetic DNA molecule, which does not occur in nature. An example of a catalytically active DNA molecule is a DNAzyme of the 10-23 family which represents a new class of anti-sense molecules developed in the 1990s. The term "10-23 family" refers to a general DNAzyme model (Sontoro & Joyce, Proc. Natl. Acad. Sci. U.S.A., 94 (1997) 4262-4266). DNAzymes of the 10-23 model - also referred to as "10-23 DNAzymes" have a catalytic domain of 15 deoxyribonucleotides, which are flanked by two substrate binding domains (e.g., WO 2005/033314). Potential advantages of DNAzymes include relatively high stability and no reliance on intracellular enzymes. Catalytically active DNA molecules such as DNAzymes found recently therapeutic application for example in the treatment of asthma (e.g., EP 3 093 022 B1). The manufacturing process of a catalytically active DNA molecule such as a DNAzyme comprises or consists of three principal process steps which are 1) synthesis, 2) cleavage and deprotection and 3) downstream purification and isolation of the DNA molecule.

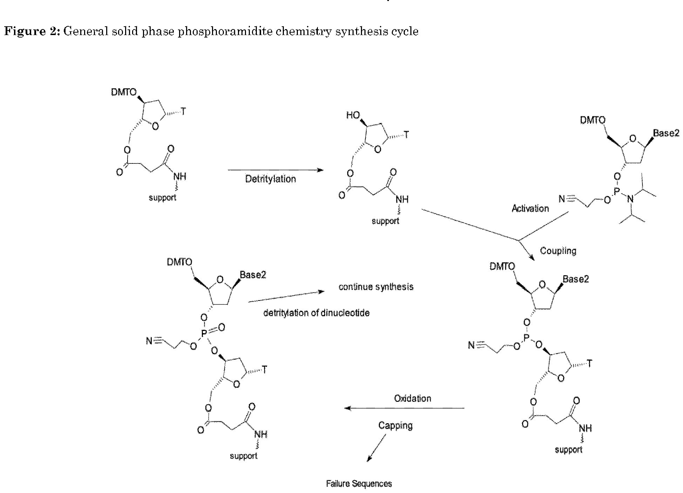

Due to the chosen synthesis and deprotection conditions of the prior art, this manufacturing process yields high amounts of ethoxyacetal-modified, depurinated species as impurities. Other process-related impurities that were detected by UPLC-MS analysis are for example longmers and incompletely deprotected species, i.e. oligonucleotides still containing the isobutyryl (ibu) protection group at the G-base (see Fig. 2).

Most of these impurities have a direct negative impact on the Watson-Crick base-pairing required for the DNAzyme activity and might therefore lower the activity of the product. In addition, these impurities lower the final purified active pharmaceutical ingredient (API) yield. Furthermore, the overall productivity of the process is low, resulting in an expensive API.

These production-specific impurities and modifications of the catalytically active DNA molecule such as loss of a nucleobase via depurination with or without subsequent formation of ethoxyacetal-modified ribose and/or incomplete deprotection of the nucleobases significantly reduce the Watson-Crick-binding of the catalytically active DNA molecule to the target mRNA which consequently significantly reduce the efficiency of the catalytically active DNA molecule. Reduced efficiency of the catalytically active DNA molecule has to be compensated by administration of increased doses which increases the risk of side effects and costs.

Moreover, the modifications and impurities of the catalytically active DNA molecule have almost identical physicochemical characteristics as the catalytically active DNA molecule and thus, can only be separated via chromatography under high loss of the catalytically active DNA molecule.

Thus, there is a high need of an oligonucleotide such as a catalytically active DNA molecule having increased activity and efficiency, respectively, which is produced with a reasonable effort and at reasonable costs.

### Summary of the invention

The present invention refers to a method for the production of a catalytically active DNA molecule such as a DNAzyme comprising the steps:
a) synthesis of the catalytically active DNA molecule on a support, wherein nucleotides comprising a nucleobase protecting group, which is for example a base-labile acyl group, are assembled in a sequential manner starting from the 3'-end to the 5'-end or from the 3'-end to the 5'-end employing a mix of an organic proton-donating activator, which is for example 0.2 to 0.45 M tetrazole or a derivative thereof such as ethylthiotetrazole (ETT), benzylthiotetrazole (BTT), dacitivity, dicyanoimidazole (DCI) or a combination thereof, and a monomeric building block amidite,
b) after completion of the synthesis cleaving the catalytically active DNA molecule from the support and the nucleobase and/or backbone protecting groups from the catalytically active DNA molecule at a temperature of 30 to 45 °C for a duration of 5 to 20 h,
c) purifying the catalytically active DNA molecule via liquid chromatography, e.g., ion exchange chromatography, and desalting, and
d) optionally isolating the catalytically active DNA molecule via freeze drying. Activator and amidite are for example set to the ratio 50:50 to 70:30. The nucleobase and/or backbone protecting group is for example a base-labile acyl group.

The support in this method is for example a solid support such as controlled pore glass (CPG) or macro-porous polystyrene (MPPS).

The nucleotide for example further comprises a 4,4'-dimethoxytrityl (DMT) group at the 5'-hydroxyl group, a beta-cyanoethyl (C-NEt) at the 3'-phosphite group or a combination thereof.

The DNAzyme is for example hgd40 comprising SEQ ID NO.1 (5 'GTGGATGGAggctagctacaacgaGTCTTGGAG).

The present invention further refers to a catalytically active DNA molecule obtainable by the method of the present invention comprising impurities in the range of < 12 wt-% referring to the total of all impurities eluting before and after the main product peak in liquid chromatography. The catalytically active DNA molecule is for example a DNAzyme, which is for example directed to GATA-3.

The catalytically active DNA molecule or the pharmaceutical composition of the present invention is for example use in a method of preventing and/or treating a human patient suffering from a GATA-3-driven disease.

The catalytically active DNA obtainable by the method of the present invention is for example for use in a method of preventing and/or treating a human patient suffering from a type-2 asthma, wherein the human patient is characterized by (i) a blood eosinophil count of 3% or more, particularly of 4% or more, more particularly of 5% or more; and/or (ii) blood eosinophil count of 350 x 10⁶ / L or more, particularly of 450 x 10⁶ / L or more; and/or (iii) fractional expiratory nitric oxide of 40 ppb or more.

Moreover, the present invention relates to a pharmaceutical composition comprising a catalytically active DNA molecule obtainable by the method of the present invention and a pharmaceutically acceptable carrier.

The pharmaceutical composition for example is for use in a method of preventing and/or treating a human patient suffering from a type-2 asthma, wherein the human patient is characterized by (i) a blood eosinophil count of 3% or more, particularly of 4% or more, more particularly of 5% or more; and/or (ii) blood eosinophil count of 350 x 10⁶ / L or more, particularly of 450 x 10⁶ / L or more; and/or (iii) fractional expiratory nitric oxide of 40 ppb or more.

The ccatalytically active DNA molecule or the pharmaceutical composition are for example administered orally, nasally, intravenously, subcutaneously, topically, rectally, parenterally, intramuscularly, intracisternally, intravaginally, intraperitoneally, intrathecally, intravascularly, locally (powder, ointment or drops) or in the form of a spray or inhalant.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### Description of the figures

**Fig. 1** shows a scheme for the synthesis of an oligonucleotide including a catalytically active DNA such as a DNAzyme.
**Fig. 2** depicts a general solid phase phophoramidite synthesis cycle.
**Fig. 3** depicts a batch comparison of a DNAzyme prepared according to the method of the present invention (X48179K1K2) and batches of DNAzymes prepared according to methods of the prior art.

### Detailed description

The present invention refers to a method for the production, e.g., scalable production, of an oligonucleotide such as a catalytically active DNA molecule, e.g., a DNAzyme. The method comprises the use of an activator such as an organic proton-donating activator and amidite for example dissolved in a dry unipolar organic solvent such as acetonitrile during the synthesis step. Further the method comprises a deprotection step at a temperature of 30 °C to 45 °C for 5 to 20 h.

The great advantage of the present invention is the production of oligonucleotides such as a catalytically active DNA molecule, e.g., a DNAzyme comprising significantly reduced impurities. This reduction in impurities results in an increase of activity, e.g., catalytic activity, of the oligonucleotide which allows reduction of the amount of the oligonucleotide in its applications. In addition, the catalytically active DNA molecule obtainable by the method of the present invention shows improved interaction, e.g., binding to its substrate which is characterized by improved kinetics.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

Commonly, the **synthesis** of oligonucleotides including a catalytically active DNA molecule such as a DNAzyme is performed by sequential solid phase chemistry employing well-established phosphodiester or phosphite triester protocols by using monomeric building blocks such as H-phosphonate and phosphoramidite building blocks, respectively. The fully-automated synthesis process is performed using commercially available computer-controlled DNA synthesis instrumentation and flow-through, fixed-bed or batch-type, stirred bed technology. For in process control and online monitoring of the ongoing process, the synthesizer contains online UV, pressure and conductivity detectors.

Briefly, these approaches comprise a solid support such as a solid support resin, functionalized with amino and/or hydroxyl moieties and a linker molecule, subsequently anchoring the 3'-most nucleoside of the oligonucleotide. By controlled chemical reactions, the desired oligonucleotide sequence is sequentially assembled, e.g., synthetically assembled, on the solid support by the stepwise addition of the respective nucleotide residues. The catalytically active DNA molecule is for example synthetically assembled in a sequential manner according to the phosphoramidite or H-phosphonate chemistry. Inter-nucleoside linkages are formed between the 3'-functional group of the incoming nucleoside and the highly-reactive 5'-hydroxyl group of the respective 5'-terminal nucleoside of the solid support-bound oligonucleotide. In the phosphoramidite approach, the inter-nucleoside linkage is a protected phosphite triester moiety, whereas in the H-phosphonate approach, it is an H-phosphonate phosphodiester moiety.

A state-of-the-art solid phase support resin is being used as starting point for the synthesis of an oligonucleotide including a catalytically active DNA molecule such as a DNAzyme (e.g., hgd40) of the present invention. The solid phase base material can either be of organic (polymeric), or inorganic nature, i.e., CPG (controlled pored glass).

The 2'-deoxy-phosphoramidite used as monomeric synthons during the solid phase synthesis carry for example an acid labile protection group at the 5'-hydroxyl function of the ribose such as a 4,4'-dimethoxytriphenylmethyl (DMT) group, and a base-labile protection group on the 3'-O-(N, N)-dialkyl-phosphite triester group such as a β-cyanoethyl (C-NEt).

Potentially reactive exocyclic amino functions of the three nucleobases adenine, cytidine, and guanine are for example protected by base-labile acyl groups to prevent undesired side-reactions and impurity-formation during the synthesis process, respectively; thymidine does not have an exocyclic amino function and therefore does not require any nucleobase protection.

As first step in the synthesis during the condensation reaction, the (N,N)-diisoalkyl-group is protonated using an organic proton-donating activator, e.g., a weak organic acid such as a Bronsted Acid, usually tetrazole or a tetrazole-derivative such as benzylthiotetrazole / 5-(Benzylmercapto)-1H-tetrazole (BTT, BMT), dicyanoimidazole (DCI) and/or 5-(Ethylthio)-1H-tetrazole (ETT). This nucleophilic attack forms for example a tetrazolide salt between the incoming monomer and the activator as a highly-reactive intermediate that completes the condensation reaction on the free 5'-hydroxyl group of the support-bound oligonucleotide chain.

The 2'-deoxy-H-phosphonate monoesters used as monomeric synthons during the solid phase synthesis carry an acid labile protection group at the 5'-hydroxyl function of the ribose such as a 4,4'-dimethoxytriphenylmethyl (DMT, dimethoxytrityl) group. There is no requirement for a phosphate protection group.

The oligonucleotide is for example assembled in a linear, multi-step solid phase synthesis and no intermediates are isolated during this process.

The synthesis cycle of an oligonucleotide such as a catalytically active DNA molecule initiates at the inverted deoxy-thymidine 3'-nucleoside, which is for example DMT protected at the 3'-hydroxy function and covalently attached to the solid phase bead for example by the 5'-hydroxy function and via a base-labile succinyl linker. Onto this nucleoside the next nucleotide in the sequence is coupled followed by consecutive 3' -> 5' elongation of the sequence of oligonucleotide such as a catalytically active DNA molecule by stepwise addition of nucleotide residues.

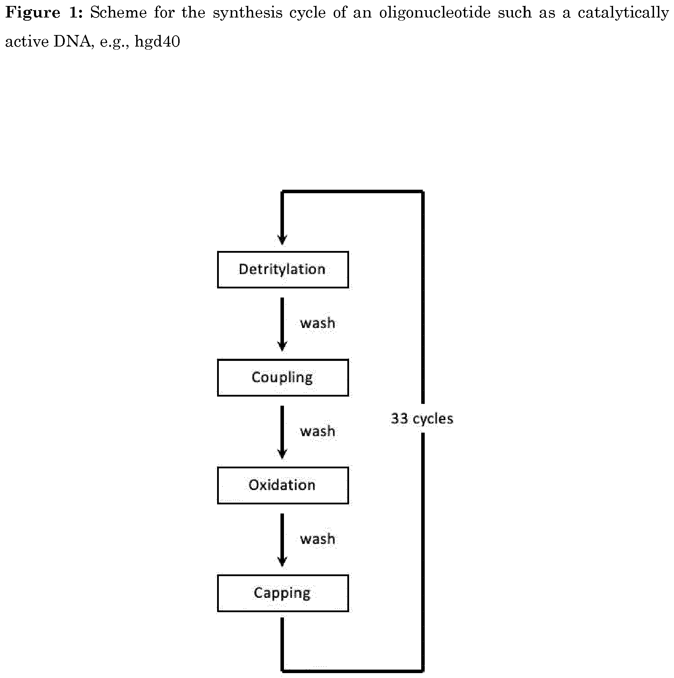

All reagents required for the addition of one nucleoside are delivered for example through the column according to a defined and computer-controlled synthesis recipe. Each coupling cycle consists of the following four primary chemical steps shown in Fig. 1.

Between each primary step for example an acetonitrile wash step is performed to remove excess reactants and reaction side-products. The oligonucleotide elongates by addition of a 3'-phosphoramidite to the highly reactive 5'-OH-terminus of the growing chain. The synthesis cycle is repeated until the synthesis of the full-length oligomer is completed according to the programmed nucleotide sequence of the catalytically active DNA molecule.

Subsequent to the synthesis of the oligonucleotide, **cleavage and deprotection** of this oligonucleotides takes place. Following the final detritylation step of the synthesis of the catalytically active DNA molecule, the β-cyanoethyl protecting groups of the bridging phosphate triester linkages are for example removed by reacting the support-attached oligonucleotide with a solution of diethylamine in acetonitrile. This β-cyanoethyl deprotection step is equally performed on the synthesiser in a controlled manner.

After completion of the DEA-treatment, the column is for example removed from the synthesiser and the contained solid support is for example dried by an inert gas such as nitrogen or argon. Successively, the oligonucleotide is released from the solid support by cleavage of the base-labile succinyl linker with concentrated ammonia in ethanol. The solid phase is then removed from the column and the oligonucleotide is successively released from the solid support for example by cleavage of the base-labile succinyl linker with a base such as concentrated ammonia or ethylamine or a mixture thereof (ammonia methylamine, AMA), in an appropriate solvent such as water or ethanol optionally at elevated temperatures for example in a range between 20°C and 70°C, between 25°C and 65°C, between 30°C and 60°C, between 35°C and 55°C or 40°C and 50°C. Higher temperatures speed the reaction up, but also increase the rate of chemical modification or degradation of the oligonucleotide including an catalytically active DNA, e.g., hgd40, leading to unwanted impurities.

This treatment also cleaves the base-labile nucleobase-protection groups. After removal of the remaining solid support beads for example by filtration, the crude product will be obtained in the respective deprotection solution, e.g., an ammonia solution. Alternatively, the deprotection solution is for example washed in a Flow Through manner for example over 10 - 120 min, 15 - 90 min or 30 - 60 min through the column at, e.g., room temperature to release the linker between oligonucleotide and solid phase bead. The oligonucleotide is then released in solution and this oligonucleotide-containing solution is optionally transferred to a deprotection step, e.g., in a deprotection vessel, and is deprotected for example by heating of the solution. After completion of the synthesis, the catalytically active DNA molecule obtainable by a method of the present invention such as a DNAzyme is for example cleaved from the solid phase support resin and the nucleobase and backbone protecting groups are successively removed using a basic aqueous solution. The temperatures are for example elevated to 30 °C to 45 °C for a duration of for example 5 to 20 h.

The manufacturing process of the oligonucleotide of the present invention ends for example with a **purification and isolation procedure.** The crude full-length oligonucleotide-containing solution obtained after cleavage and deprotection contains a variety of product- and process-related impurities, such as failure sequences (n-1 species, shortmers), sequences with additional bases (n+x species, longmers), chemically modified products derived from the incomplete cleavage of the protecting groups, and base-modified compounds, respectively. Furthermore, during the acidic detritylation step, depurination of nucleotides may occur and lead to the generation of abasic products, missing one or more purine base. Upon treatment with basic aqueous solutions e.g. during the cleavage with ammonia these abasic products are partially degraded into shorter (n-x)- fragments.

Any of these potential impurities can be separated during the purification for example by liquid chromatography such as Ion Exchange (IEX) Liquid Chromatography.

The crude DMT-off product obtained from the cleavage procedure is for example diluted and loaded at a defined concentration onto a chromatography column packed with a strong anion exchange resin. The separation of the full-length product from by-products is achieved by a gradient of sodium chloride in an aqueous alkaline solution. During the gradient separation, fractions of the effluent containing oligonucleotide are collected and successively analysed by UPLC-MS.

Fractions that meet the given specification criteria are then pooled and analyzed again by IEX-HPLC to confirm the result of the pooling.

These combined fractions contain excess salt from the gradient elution and are for example submitted to Tangential Flow Filtration (TFF). If necessary, the pH of the solution is adjusted during the buffer exchange step, and the filtered and concentrated product is then freeze dried to yield the final active pharmaceutical ingredient (API) as amorphous powder or cake. The API is harvested and finally stored in sterile HDPE bottles with screw cap (Nalgene®) at -20°C.

**Table 1: Product purity and levels of impurities pre- and post-product peak of hgd40 batches in comparison to the new process according to the present invention.**

| **List of Batches hgd40** | Batch-ID | Batch-ID (Biospring) | Year | rrt <1.00 (%) | flp (%) | rrt >1.00 (%) |
|---|---|---|---|---|---|---|
| Tox Batch 1 | 138976 | A05 | 2009 | 15.66 | 61.05 | 19.74 |
| Tox Batch 2 | 158602 | A16 | 2010 | 13.21 | 75.64 | 7.01 |
| Clinical Batch 1 | 161713 | GMP_A11 | 2011 | 5.63 | 84.36 | 6.12 |
| Clinical Batch 2 | 200293 | GMP_BA | 2013 | 5.31 | 83.75 | 8.94 |
| Tox Batch 3 | 254936 | A120C | 2017 | 3.07 | 84.35 | 9.81 |
| Tox Batch 4 | 257848 | A191 | 2018 | 4.68 | 79.89 | 9.20 |
| | | | | | | |
| 10gm new process | X48179K1K2 | | 2019 | 5.70 | 88.73 | 4.02 |

Due to the chosen synthesis and deprotection conditions of the prior art, this manufacturing process yields high amounts of ethoxyacetal-modified, depurinated species as impurities. Other process-related impurities that were detected by UPLC-MS analysis are longmers and incompletely deprotected species, i.e. oligonucleotides still containing the isobutyryl (ibu) protection group at the G-base (see Fig. 2).

Most of these impurities have a direct negative impact on the Watson-Crick base-pairing required for the DNAzyme activity and might therefore lower the activity of the product. In addition, these impurities lower the final purified active pharmaceutical ingredient (API) yield. Furthermore, the overall productivity of the process is low, resulting in an expensive API.

**Table 2: Detailed analysis showing the four most dominant process-related impurities in comparison to the new process according to the present invention.**

| **List of Batches hgd40** | Batch-ID | Batch-ID (Biospring) | Year | Ethoxyaceta I-Impurity | +ibu | (n+1) | (n-1) |
|---|---|---|---|---|---|---|---|
| Tox Batch 1 | 138976 | A05 | 2009 | 4.79 | 4.19 | 1.10 | 4.96 |
| Tox Batch 2 | 158602 | A16 | 2010 | 5.63 | 2.40 | 0.88 | 1.69 |
| Clinical Batch 1 | 161713 | GMP_A11 | 2011 | 4.86 | 1.48 | 0.78 | 2.39 |
| Clinical Batch 2 | 200293 | GMP_BA | 2013 | 4.42 | 1.03 | 1.22 | 0.57 |
| Tox Batch 3 | 254936 | A120C | 2017 | 8.53 | 0.00 | 1.27 | 1.86 |
| Tox Batch 4 | 257848 | A191 | 2018 | 2.12 | 4.94 | 2.14 | 0.79 |
| | | | | | | | |
| 10gm new process | X48179K1K2 | | 2019 | 0.00 | 0.00 | 0.97 | 2.90 |

As can be deduct from Table 2, a significant amount (2.12% - 8.53%) of the ethoxyacetal impurity is being generated in each batch. This impurity arises during the cleavage and deprotection step using ethanolic ammonia solution at elevated temperatures (55°C) and is clearly process-related. The impurity is difficult to remove during HPLC purification and cannot form Watson-Crick base-pairing, thus lowers the activity of the catalytically active DNA molecule such as a DNAzyme.

Residual isobutyryl protection groups (ibu) on the G-nucleobase can be detected at levels between 0.00% and 4.94% in each batch. This impurity is related to an incomplete deprotection of the G-nucleobase during the ammonia-treatment. It is difficult to remove during HPLC purification and blocks Watson-Crick base-pairing, thus lowers the activity of the catalytically active DNA molecule such as a DNAzyme.

The (n+1) impurity represents a heterogenous group of impurities with one additional base at a random position in the molecule. Depending on the position within the molecule, the additional base might distract proper binding of the catalytically active DNA molecule such as a DNAzyme to the target mRNA, thus lowering the activity. The double-coupling happens because of uncontrolled coupling reaction. It can be found at levels between 0.78% and 2.14% in all batches. During purification, the impurity elutes close to the main peak and is very difficult to remove.

The (n-1) impurity represents a heterogenous group of impurities missing one base at a random position in the molecule. Depending on the position within the molecule, the missing base might distract proper binding of the catalytically active DNA molecule such as a DNAzyme to the target mRNA, thus lowering the activity. The missing base can be related to either incomplete coupling of the incoming base (non-optimized coupling conditions), or incomplete oxidation and related strand cleavage during the detritylation step. It can be found at levels between 0.57% and 4.96% in all batches. During purification, the impurity elutes close to the main peak and is extremely difficult to remove.

The crude yield (before purification) obtained with this process was measured between 2.57 and 3.41 gm / mmol synthesis scale. As the catalytically active DNA molecule such as the DNAzyme hgd40 has a molecular mass of 10603 Da and thus a theoretical yield of 10.6 gm / mmol synthesis scale, the observed yield before purification is between 24.25% and 32.17%. During purification and downstream another 50% yield loss can be expected. Total overall yield therefore calculates between 12.13% and 16.85%.
Following the in-depth analysis of all available DNAzyme hgd40-batches through high-resolution UPLC-MS, relevant individual process steps were optimized to minimize the process-related impurity burden.

**Table 3: Exemplary high-resolution impurity profile of a single batch (clinical batch 2) manufactured in 2013 according to prior art. RRT is the abbreviation for "Relative Retention Time", FLP for "Full Length Product" and ID for "Identification".**

| **hgd40_Batch200293 (Clinical Batch 2, GMP, prior art, purified)** | | | | |
|---|---|---|---|---|
| **RRT to FLP** | **Relative Peak Area (HPLC-UV) [%]** | **MW (Average) [Da]** | **Δ Mass** | **ID** |
| 0.725 | 0.82 | 9310.63 | -1292.26 | -5'-d(GTGG) |
| | | 9639.69 | -963.20 | -5'-d(GTG) |
| 0.796 | 0.93 | 9968.75 | -634.14 | -5'-d(GT) or -3'-d(G-iT) |
| 0.882 | 0.72 | 10635.86 | 32.97 | *2* A -> G Exchange ?* |
| 0.897 | 1.39 | 9735.67 | -867.23 | -3'd(AG-iT) +P |
| | | 10635.86 | 32.97 | *2* A -> G Exchange ?* |
| 0.951 | 0.57 | 10272.80 | -330.09 | -dG |
| 0.971 | 0.88 | 10618.85 | 15.96 | *A -> G Exchange ?* |
| 1.000 | 83.75 | 10602.89 | 0.00 | flp |
| 1.217 | 0.82 | 10377.79 | -225.11 | iT+P (3') |
| | | 10931.92 | 329.03 | +dG |
| 1.349 | 0.397 | 10890.91 | 288.01 | +dC |
| | | 10906.91 | 304.02 | +dT |
| | | 10915.93 | 313.03 | +dA |
| | | 10352.77 | -250.12 | *Unknown* |
| 1.596 | 0.88 | 10512.86 | -90.03 | depur A + ethoxyacetal |
| 1.610 | 0.54 | 10512.86 | -90.03 | depur A + ethoxyacetal |
| 1.621 | 2.12 | 10512.87 | -90.03 | depur A + ethoxyacetal |
| 1.630 | 0.88 | 10496.87 | -106.03 | depur G + ethoxyacetal |
| | | 10512.86 | -90.03 | depur A + ethoxyacetal |
| | | 10696.89 | 94.00 | *Unknown* |
| 1.654 | 2.28 | 10643.87 | 40.98 | *G -> N2-acetyl-2,6-diamino purine Exchange* |
| | | 10800.94 | 198.04 | *Unknown* |
| | | 10778.93 | 176.04 | *Unknown* |
| 1.686 | 1.03 | 10809.98 | 207.09 | +3*ibu |
| | | 11199.48 | 596.59 | *Unknown* |

Table 3 above describes exemplary the impurity profile after purification for hgd40 batch #200293, manufactured in 2013 using the established manufacturing process of the present invention. As depictured in Table 1, this batch was released with a purity of 83.75% with a crude yield of for example 4.77 gm/mmol synthesis scale. As hgd40 has a molecular mass of 10603 Da and thus, a theoretical yield of for example 10.6 gm/mmol synthesis scale, the observed yield before purification calculates for example to 45.00 %. The pre-product impurities peaks calculate to 5.31%, and post product impurities peaks add up to 8.94%. The final yield at release of the molecule is calculated for example to 2.47 gm/mmol or 23.30 % calculated against the theoretical yield.

The method of the present invention is based on any of the previously described methods, but significantly reduces or even eliminates impurities such as (n+1) impurity, (n-1) impurity or combinations thereof. A catalytically active DNA molecule such as a DNAzyme, obtainable by the method of the present invention comprises significantly reduced total impurities for example total impurities in the range of < 20 %, < 15 %, < 12%, < 10 % or < 5 % referring to all impurities eluting before and after the main product peak via liquid chromatography such as analytical HPLC or FPLC.

The reduced impurities of the catalytically active DNA molecule do not only improve for example the catalytic activity, but also the substrate binding of the catalytically active DNA molecule, i.e., the interaction of the catalytically active DNA molecule with its substrate. These improvements result for example in an improved of the potency of the catalytically active DNA molecule compared to a catalytically active DNA molecule of the prior art.

The method of the present invention comprises for example Controlled Pore Glass (CPG) or macroporous polystyrene (MPPS) as a polymeric resin in the synthesis of the oligonucleotide. The resin loading is for example in the range of 10 to 500 µmol/gm, 50 to 450 µmol/gm, 100 to 400 µmol/gm, 150 to 350 µmol/gm, 200 to 300 µmol/gm or 60 to 120 µmol/gm.

Optionally the method of the present invention comprises the use of an activator and/or of amidite. The activator is for example an acid such as an organic acid, e.g., an organic Bronsted Acid such as tetrazole or a derivative thereof such as ethylthiotetrazole (ETT), benzylthiotetrazole (BTT/BMT) or dicyanoimidazole (DCI) which is for example dissolved in a dry unipolar organic solvent such as acetonitrile. The activator such as the Bronsted Acid may comprise an additive such as a basic compound for example N-methyl imidazole, e.g., in a concentration of 0.05 to 0.2 M. The concentration of the activator such as tetrazole or a derivative thereof, e.g., ETT, BTT/BMT or DCI is for example 0.1 M to 1 M, 0.2 M to 0.8 M, 0.3 M to 0.6 M or 0.5 M. The amidite concentration is for example 0.1 to 0.25 M. The amidite is for example dissolved in a dry unipolar organic solvent such as acetonitrile. Amidite equivalents are for example 0.5 to 5.0 eq, 1.0 to 4.0 eq, 1.5 to 3.5 eq or 1.2 to 3.0 eq. The ratio of the activator : amiditein the method of the present invention is for example 10 : 90, 20 : 80, 30 : 70, 40 : 60, 50 : 50, 60 : 40, 70 : 30, 80 : 20 or 90 : 10, or 10 : 90 to 90 : 10, 20 : 80 to 80 : 20, 30 : 70 to 70 : 30, 40 : 60 to 60 : 40 or 50 : 50 to 70 : 30.

Optionally the method of the present invention further uses a capping reagent which is for example an acid anhydride such as acetic acid anhydride, e.g., dissolved in a dry unipolar organic solvent such as acetonitrile. The amount of the acid anhydride such as acetic acid anhydride is for example 3 to 100 %, 5 to 50 %, 10 to 40 %, 20 to 30 % or 10 to 30 %.

In the cleavage and deprotection step for example a basic aqueous solution is used such as concentrated aqueous ammonia solution. The duration of the cleavage and deprotection step is for example 1 to 24 h, 5 to 20 h, 10 to 15 h or 8 to 16 h. The temperature of the cleavage and deprotection step is for example 10 to 60 °C, 15 to 55 °C, 20 to 50 °C, 30 to 45 °C or 35 to 40 °C.

The purification resin used in the purification step of the present invention are for example porous hydrophilic polymer beads which are modified with a strong anion exchange group. An example of such polymer is TSKGel SuperQ 5PW 20.

In the purification step for example two different buffers are used which are for example alkali hydroxides such as sodium hydroxide alone or a combination of an alkali hydroxides and an alkali halide. A first buffer comprises or consists of for example 1 to 30 mM, 5 to 25 mM, 10 to 20 mM or 5 to 25 mM sodium hydroxide in water; a second buffer comprises or consists of for example 1 to 30 mM, 5 to 25 mM, 10 to 20 mM or 5 to 25 mM sodium hydroxide in water and 0.5 to 2.0 NaBr, 1.0 to 1.8 NaBr or 1.5 NaBr.

All the above mentioned parameters can be combined with each other in the different amounts and concentrations, respectively.

An example of the materials and method steps used in the method of the present invention in comparison to the materials and method steps used in common prior art methods is shown in the following Table 4:

**Table 4: CPP of prior art methods in comparison to CPP of the method of the present invention**

| **Process Step** | | **Prior art method** | **Method of the present invention** |
|---|---|---|---|
| ***Synthesis*** | Synthesis Resin | Polymeric Resin | CPG (Controlled Pored Glass) |
| | Resin Loading | 250 µmol/gm | 60 - 120 µmol/gm |
| | Activator | 0.25M Activator 42 in acetonitrile | 0.3 - 0.6M Ethylthiotetrazole (ETT) in acetonitrile |
| | Amidite Concentration | 0.1M in acetonitrile | 0.1 - 0.2M in Acetonitrile |
| | Amidite Equivalents | 1.75 eq | 1.2 - 3.0 eq |
| | Activator : Amidite Ratio | 60:40 | 50:50 - 70:30 |
| | Capping Reagent | 5% (v/v) tert-Butylphenoxyacetic acid anhydride in acetonitrile | 10 - 30% (v/v) Acetic acid anhydride in acetonitrile |
| ***Cleavage and Deprotection*** | Cleavage Reagent | Conc. aq. ammonia solution in ethanol (3:1, v/v) | Conc. aq. ammonia solution |
| | Cleavage & Deprotection | 17h, 55 °C | 8 - 16h, 30 - 45 °C |
| ***Purification*** | Purification Resin | GE Healthcare Source 30Q | Tosoh TSKGel SuperQ 5PW 20 |
| | Purification Buffer | Buffer A: 10 mM sodium hydroxide in water | Buffer A: 5 - 25 mM sodium hydroxide in water |
| | | Buffer B: 10 mM sodium hydroxide, 1.5 M NaCl in water | Buffer B: 5 - 25 mM sodium hydroxide, 1.0 - 1.8 M NaBr in water |
| | Purification Conditions | Gradient over 10 CV, room-temperature | Gradient over 10 - 30 CV, 30 - 50 °C |

All process changes described and listed above are Critical Process Parameters (CPP) for DNA synthesis and can be directly linked to the observed impurity profile as shown in Tables 3 and 4.

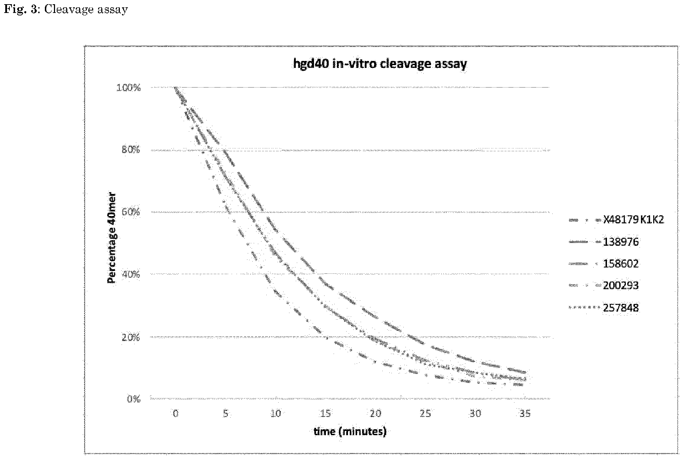

A catalytically active DNA molecule of the present invention obtainable by the method of the present invention is for example a DNAzyme directed at GATA-3. The sequence of this DNAzyme is for example selected from the sequences hgd1 to hgd70 of WO 2005/033314 (see Figure 3 of WO 2005/033314), particularly selected from the sequences hgd11, hgd13, hgd17 and hgd40, more particularly the sequence of hgd40 (5'-GTGGATGGAggctagctacaacgaGTCTTGGAG; SEQ ID NO:1), i.e., the DNAzyme comprises or consists of these sequences.

The DNAzyme "hgd40" comprises or consists of for example 34 bases with the sequence 5'-GTGGATGGAggctagctacaacgaGTCTTGGAG. The nine bases at both the 3' and 5' region form two binding domains, which highly specifically bind to the target mRNA of GATA-3. The central core of the molecule represents the catalytic domain which accounts for cleavage of the target following binding of hgd40 to the GATA-3 mRNA. The drug substance hgd40 is characterized by high bioactivity and bioavailability at the site of drug delivery by inhalation.

The catalytically active DNA molecule of the present invention such as the DNAzyme hgd40 is for example comprised in a formulation that can be administered to a patient either administered orally, nasally, intravenously, subcutaneously, topically, rectally, parenterally, intramuscularly, intracisternally, intravaginally, intraperitoneally, intrathecally, intravascularly, locally (powder, ointment or drops) or in the form of a spray or inhalant. The active component is for example mixed under sterile conditions with a physiologically acceptable excipient and possible preservatives, buffers or propellants, depending on requirements.

The DNAzyme hgd40 is for example comprised in a formulation for inhalation or dissolved in PBS.

The catalytically active DNA molecule such as a DNAzyme obtainable by a method of the present invention is for example used in a method of the prevention and/or treatment of a GATA-3-derived disease, disorder or condition. Such disease, disorder or condition is any disease, disorder or condition in which GATA-3 is upregulated in a cell compared to the normal level in a cell. The cell is any cell expressing GATA-3 for example an immune cell. The upregulation of GATA-3 is for example associated with the initiation, influence of and/or escalation of a pathological process leading for example to a disease, disorder or condition outbreak, development of symptoms and/or progression of the disease, disorder or condition. Such disease is for example a type-2 inflammation or disease, e.g., a TH-2-driven disease, disorder or condition such as TH2-derived asthma.

### Examples

The following examples illustrate different embodiments of the present invention, but the invention is not limited to these examples.

### Example 1: Preparation of a high-resolution impurity profile of a small scale single batch

The crude yield (before purification) of a hgd40 DNAzyme obtained with the method of the present invention was measured at 5.91 gm / mmol synthesis scale. As hgd40 has a molecular mass of 10603 Da and thus, a theoretical yield of 10.6 gm / mmol synthesis scale, the observed yield before purification calculates to 55.75% (+9.75% in comparison to the previous process). During purification and downstream a yield of 67.8% was observed. The pre-product impurities peaks calculate to 5.70% (+0.39%), and post product impurities peaks add up to 4.02% (-4.92%). Two impurities completely disappeared (ethoyxacetal and isobutyryl-modification) due to the process changes, and one new impurity was detected at low percentage (CNET). Total overall yield therefore calculates to 37.5% (+14.20% in comparison to the previous process).

**Table 5 shows an exemplary high-resolution impurity profile of a small scale single batch manufactured in 2019, using the method of the present invention:**

| **HGD40_X48179K1K2 (non GMP, 10 gm batch, synthesized in 2019)** | | | | |
|---|---|---|---|---|
| **Peak ID** | **Relative Peak Area (HPLC-UV)** | **MW (Average)** | **Δ Mass** | **ID** |
| | **[%]** | **[Da]** | | |
| 1 | 0.24 | 5357.09 | -5244.29 | Unknown |
| | | 7441.76 | -3159.62 | Unknown |
| | | 8692.02 | -1909.36 | -5'-d(GTGGAT) |
| 2 | 0.23 | 8996.05 | -1605.33 | -5'-d(GTGGA) |
| 3 | 0.33 | 9310.10 | -1291.28 | -5'-d(GTGG) |
| 4 | 0.70 | 9639.13 | -962.25 | -5'-d(GTG) |
| 5 | 0.57 | 9968.18 | -633.20 | -5'-d(GT) or -3'-d(G-iT) |
| 6 | 0.00 | n/a | n/a | *n*/*a* |
| 7 | 0.37 | 10272.22 | -329.16 | -dG |
| | | 10297.20 | -304.18 | -dT |
| 8 | 0.82 | 10272.21 | -329.17 | -dG |
| 9 | 0.24 | 10273.23 | -328.15 | -dG |
| | | 10288.23 | -313.15 | -dA |
| | | 8692.02 | -289.14 | -dC |
| 10 | 0.45 | 10272.21 | -329.17 | -dG |
| | | 10602.27 | -0.73 | FLP |
| | | 10636.26 | +34.88 | Unknown |
| 11 | 88.73 | 10601.38 | -1.62 | FLP |
| 12 | 0.40 | 10602.32 | -0.68 | FLP |
| 13 | 1.94 | 10601.32 | -1.68 | FLP |
| | | 10654.32 | +53.00 | CNET |
| | | 10915.35 | +313.97 | +dA |
| | | 10931.34 | +329.96 | +dG |
| 14 | 0.71 | 9735.11 | -866.26 | -3'-d(AG-iT) +P |
| 15 | 0.33 | 10049.17 | -552.21 | -5'-d(GT) +P or -3'-d(G-iT) +P |
| 16 | 0.51 | 10377.20 | -224.18 | -3'-d(iT) +P |
| 17 | 0.24 | 10353.17 | -248.21 | Unknown |

### Example 2: Cleavage Assay

The activity of DNAzyme hgd40 is based on structure formation in solution, so a functional assay is feasible to determine on-target efficacy. Therefore, a functional *in-vitro* cleavage assay was developed to monitor the time-dependent cleavage activity of different hgd40-batches.

Hgd40 possesses a central 23 base catalytic domain flanked on both sides by GATA-3 mRNA specific, five nucleotide (nt) long binding arms. As assay target, a 40nt RNA sequence, corresponding with the endogenous mRNA GATA-3 target region of hgd40 was designed and manufactured synthetically. The 40nt RNA would be specifically cleaved by hgd40 into 17nt and 23nt fragments. These fragments can be well separated by denaturing HPLC. The cleavage kinetics of hgd40 were analyzed at different time points by denaturing ion-pairing reversed-phase high-performance liquid chromatography (IP-RP-HPLC).

**Fig. 3** shows the cleavage assay for five batches in total. Batch X48179K1K2 has been manufactured according to the method of the present invention; Batches 138976, 158602, 200293, 257848 have been manufactured according to a method of the prior art. Results have been normalized for hgd40 content in the solution and are presented in **Table 6** below.

**Table 6: Results of the cleavage assay**

| Time/Batch | **X48179K1K2** | **138976** | **158602** | **200293** | **257848** | **Δ amount 40mer** |
|---|---|---|---|---|---|---|
| | % 40mer | % 40mer | % 40mer | % 40mer | % 40mer | |
| 0 min | 100.00 | 100.00 | 100.00 | 100.00 | 100.00 | 0.00% |
| 5 min | 61.90 | 78.98 | 70.92 | 72.19 | 70.97 | 9.02 - 17.08% |
| 10 min | 34.58 | 54.15 | 46.90 | 45.62 | 46.42 | 11.04 - 19.57% |
| 15 min | 19.54 | 37.00 | 29.57 | 29.81 | 29.32 | 9.78 - 17.46% |
| 20 min | 11.84 | 26.14 | 19.46 | 18.67 | 18.43 | 6.59 - 14.30% |
| 25 min | 7.81 | 17.24 | 12.37 | 12.09 | 11.32 | 3.51 - 9.39% |
| 30 min | 5.10 | 11.85 | 8.39 | 7.27 | 8.43 | 3.33 - 6.75% |
| 35 min | 4.60 | 8.48 | 5.92 | 5.93 | 6.64 | 2.04 - 3.82% |

Table 6 indicates that hgd40 manufactured according to the method of the present invention has initially higher activity compared to the other batches. The increase in activity is directly related to the decrease of 40mer cleavage template. The method of the present invention shows an initial higher activity between 9.02 and 17.68%. The numbers decrease over time, as the template is slowly being digested with a plateau at 25 - 30 min for the method of the present invention and 35 minutes for the method of the prior art. Hgd40 manufactured according to the method of the present invention shows a faster cleavage kinetic than the old batches.

## Claims

1. Method for the production of a catalytically active DNA molecule comprising the steps:
a) synthesis of the catalytically active DNA molecule on a support, wherein nucleotides comprising a nucleobase protecting group are assembled in a sequential manner starting from the 3'-end to the 5'-end or from the 3'-end to the 5'-end employing a mix of an organic proton-donating activator, which is for example 0.2 to 0.45 M tetrazole or a derivative thereof such as ethylthiotetrazole (ETT), benzylthiotetrazole (BTT) or dicyanoimidazole (DCI) and a monomeric building blockamidite,
b) after completion of the synthesis cleaving the catalytically active DNA molecule from the support and the nucleobase and/or backbone protecting groups from the catalytically active DNA molecule at a temperature of 30 to 45 °C for a duration of 5 to 20 h,
c) purifying the catalytically active DNA molecule via liquid chromatography and desalting, and
d) optionally isolating the catalytically active DNA molecule via freeze drying.

2. Method according to claim 1, wherein the support is a solid support such as controlled pore glass (CPG) or macroporous polystyrene (MPPS).

3. Method according to claim 1 or 2, wherein the nucleobase and/or backbone protecting group is a base-labile acyl group.

4. Method according to any one of claims 1 to 3, wherein the nucleotide further comprises a 4,4'-dimethoxytrityl (DMT) group at the 5'-hydroxyl group, a beta-cyanoethyl (C-Net) at the 3'-phosphite group or a combination thereof.

5. Method according to any one of claims 1 to 4, wherein the activator is 0.2 to 0.45 M Tetrazole or a derivative thereof such as ethylthiotetrazole (ETT), benzylthiotetrazole (BTT/BMT), dicyanoimidazole (DCI) or a combination thereof.

6. Method according to any one of claims 1 to 5, wherein the ratio of activator : amidite is 50:50 to 70:30.

7. Method according to any one of claims 1 to 4, wherein the catalytically active DNA molecule is a DNAzyme.

8. Method according to claim 5, wherein the DNAzyme is hgd40 comprising SEQ ID NO.1 (5'-GTGGATGGAggctagctacaacgaGTCTTGGAG).

9. Catalytically active DNA molecule obtainable by the method according to any one of claims 1 to 4 comprising total impurities in the range of < 12 wt-% referring to the total of all impurities eluting before and after the main product peak in liquid chromatography.

10. Catalytically active DNA molecule according to claim 9, wherein the catalytically active DNA molecule is a DNAzyme, which is for example directed to GATA-3.

11. Catalytically active DNA molecule according to claim 9 or 10, wherein the DNAzyme is hgd40 comprising SEQ ID NO.1 (5'-GTGGATGGAggctagctacaacgaGTCTTGGAG).

12. Pharmaceutical composition comprising a catalytically active DNA molecule according to any one of claims 9 to 11 and a pharmaceutically acceptable carrier.

13. Catalytically active DNA molecule according to any one of claims 9 to 11 or pharmaceutical composition according to claim 12 for use in a method of preventing and/or treating a human patient suffering from a GATA-3-driven disease.

14. Pharmaceutical composition according to claim 12 or 13, or catalytically active DNA molecule according to any one of claims 9 to 12, for use in a method of preventing and/or treating a human patient suffering from a type-2 asthma, wherein the human patient is **characterized by** (i) a blood eosinophil count of 3% or more, particularly of 4% or more, more particularly of 5% or more; and/or (ii) blood eosinophil count of 350 x 10⁶ / L or more, particularly of 450 x 10⁶ / L or more; and/or (iii) fractional expiratory nitric oxide of 40 ppb or more.

15. Catalytically active DNA molecule for use according to claim 13 or pharmaceutical composition according to claim 14, wherein the catalytically active DNA molecule or the pharmaceutical composition is administered orally, nasally, intravenously, subcutaneously, topically, rectally, parenterally, intramuscularly, intracisternally, intravaginally, intraperitoneally, intrathecally, intravascularly, locally (powder, ointment or drops) or in the form of a spray or inhalant.
